Europäisches Patentamt

(19) European Patent Office

Office européen des brevets

(11) Publication number: **0 102 328**
**B1**

(12) **EUROPEAN PATENT SPECIFICATION**

(45) Date of publication of patent specification: **14.10.87**

(21) Application number: **83830149.7**

(22) Date of filing: **20.07.83**

(51) Int. Cl.⁴: **C 23 C 14/34,** A 61 F 2/00

(54) **Method of manufacturing a prosthetic device having a coating of bio-compatible carbonaceous material and a prosthetic device having such a coating.**

(30) Priority: **03.08.82 IT 6797882**

(43) Date of publication of application:
**07.03.84 Bulletin 84/10**

(45) Publication of the grant of the patent:
**14.10.87 Bulletin 87/42**

(84) Designated Contracting States:
**AT BE CH DE FR GB IT LI LU NL SE**

(56) References cited:
**EP-A-0 029 787
DE-A-2 020 804
DE-A-3 116 040
FR-A-2 364 273
FR-A-2 399 237
US-A-3 825 957
US-A-3 952 334**

**"Handbook of Thin Film Technology" by L.I. Maissel (1979), page 4-2**

(73) Proprietor: **SORIN BIOMEDICA S.p.A.
Strada per Crescentino 31
I-13040 Saluggia (Vercelli) (IT)**

(72) Inventor: **Vallana, Franco
Via Mazzini 38
I-10123 Torino (IT)**
Inventor: **Arru, Pietro
Via San Secondo 62
I-10128 Torino (IT)**

(74) Representative: **Bosotti, Luciano et al
c/o Jacobacci-Casetta & Perani S.p.A.
Via Alfieri, 17
I-10121 Torino (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 102 328 B1

## Description

The present invention relates to prosthetic devices and relates particularly to manufacturing prosthetic devices including an element having a coating of biocompatible carbonaceous material applied by cathode sputtering.

Such a prosthetic device is known from EP—A—0 029 787.

The application of such a coating gives the prosthetic device good characteristics of biocompatibility which make it possible to implant the device safely and permanently in a living body.

According to known art, the coating of biocompatible carbonaceous material is normally applied to the prosthetic device by the pyrolysis of carbon-containing materials such as, for example, methane, propane, butane or other hydrocarbons. The pyrolytic method of deposition is carried out at high temperatures typically within the range of 1300°C to 2300°C and, in any case, at temperatures above 800°C.

The need to operate at such temperatures means that coatings of biocompatible carbonaceous material can be applied only to substrates which are able to withstand the deposition temperature.

EP—A—0 029 787 discloses use of sputtering — i.e. a low-temperature coating technique — to form an insulating, refractory, amorphous and non-porous coating of a biocompatible compound including carbons on a rigid prosthesis such as buccal and/or bone prosthesis having a metallic armature.

The object of the present invention is to provide a method for manufacturing prosthetic devices such as heart valves and vascular prostheses, which in addition to a high degree of biocompatibility (haemocompatibility) show an intrinsic capability of adapting themselves resiliently to the implant environment.

According to the present invention, this object is achieved by means of a method of manufacturing a prosthetic device including an element having a coating of biocompatible carbonaceous material applied by cathode sputtering having the characteristics set forth in Claim 1 for the contracting states AT, BE, CH, FR, GB, IT, LI, LU, NL and SE.

A method of forming a biocompatible coating of a carbonaceous material having a certain degree of similarity to the method of the present invention is disclosed in DE—A—31 16 040, which is not comprised in the prior art according to Art. 54 EPC. A separate set of claims is attached for the contracting state DE.

The invention will now be described, purely by way of non-limiting example, with reference to the appended drawings, in which:

Figure 1 illustrates apparatus for carrying out the method according to the invention,

Figure 2 illustrates different apparatus for carrying out the method,

Figure 3 is a perspective view of a prosthetic device according to the invention,

Figure 4 is a perspective view of one of the elements of the device of Figure 3, and

Figure 5 illustrates another prosthetic device according to the invention.

In the present specification and the following claims the term "cathode sputtering" has been used to indicate generally the physical phenomena whereby, during the passage of an electric current (discharge) through a rarefied gas housed in a chamber (bell), the material of one of the electrodes (target) used for generating the discharge is projected into the chamber and is deposited on an object (substrate) located within the chamber itself.

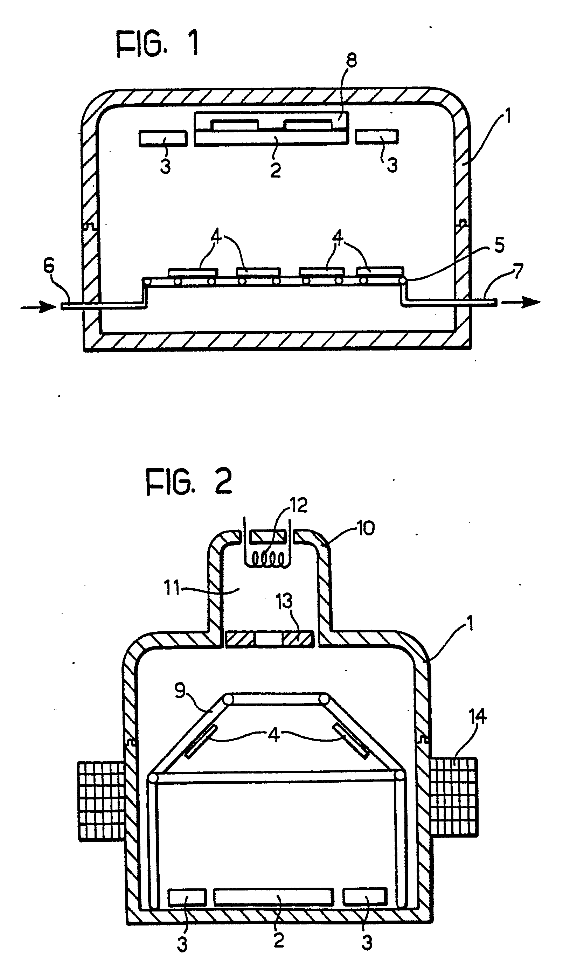

In Figures 1 and 2, two chambers (bells) are indicated 1 within which a high vacuum may be formed in known manner and into which a rarefied gas, for example argon, is introduced.

Figure 1 shows a cathode and an anode, indicated 2, 3 respectively, between which an electric discharge is established by supply means not illustrated.

The cathode 2 is constituted by a carbonaceous material selected from the group consisting of graphite, glassy carbon and carbon with a turbostratic structure.

As a result of the electric discharge established between the anode 3 and the cathode 2, the carbonaceous material of the cathode 2 is sputtered within the bell-shaped chamber 1 and is deposited as a coating of carbonaceous material on the surface of elements 4 to be coated, located on a support 5 which may be polarizable.

The elements 4 to be coated are constituted by prosthetic devices or parts of prosthetic devices, such as for example, components of cardiac-valve prostheses or vascular prostheses.

In the embodiment illustrated, the support 5 is located below the cathode (target) 2 and has passages within it for a coolant fluid which is introduced into the bell through an inlet duct 6 and leaves the bell 1 through a discharge duct 7.

The arrangement of the support 5 beneath the target 2 facilitates the introduction of the support 5 and the element 4 located thereon into the chamber 1 and their removal therefrom through apertures (not illustrated) closed by sealed doors and located in the lower part of the side walls of the bell.

Finally, a magnet 8 is shown which is intended, in known manner, to generate a magnetic field in the zone occupied by the cathode 2 and the anode 3 in order to deflect the free electrons generated in the process within the bell 1 into an approximately helical or cycloidal path, increasing the efficiency of the bombarding action on the target 2 achieved by the ions Ar$^+$ by increasing the density of the plasma.

In the cathode sputtering device illustrated in Figure 2, the cathode (target) 2 and the annular anode 3 are located on the base of the chamber 1.

The prosthetic devices 4 to be coated are mounted on a support structure 9 in such a position that the elements 4 face the target 2.

The upper wall of the chamber 1 has a raised part 10 defining a prechamber 11 within which is a filament 12 for generating electrons.

An auxiliary anode indicated 13, substantially in the form of an iris, is intended to accelerate the electrons produced by the filament 12 within the prechamber 11 so as to form a plasma beam directed at the target 2.

This beam is collimated by a winding (coil) 14 located outside the chamber 1 and having a generally cylindrical form.

As is known, the use of the electron source 12 and the auxiliary anode 13 allows the cathode sputtering to be carried out at lower pressures with a considerable improvement in the results obtainable.

In both the devices illustrated, the temperature within the bell 1 may be kept at about atmospheric temperature since the sputtering of the target material 2 and its diffusion within the bell 1 is caused by the transfer of kinetic energy from the ions Ar$^+$ to the material of the target 2. The sputtering process is thus intrinsically a "cold" process which may be carried out at temperatures substantially less than the temperature needed for depositing a layer of biocompatible carbonaceous material by means of pyrolysis.

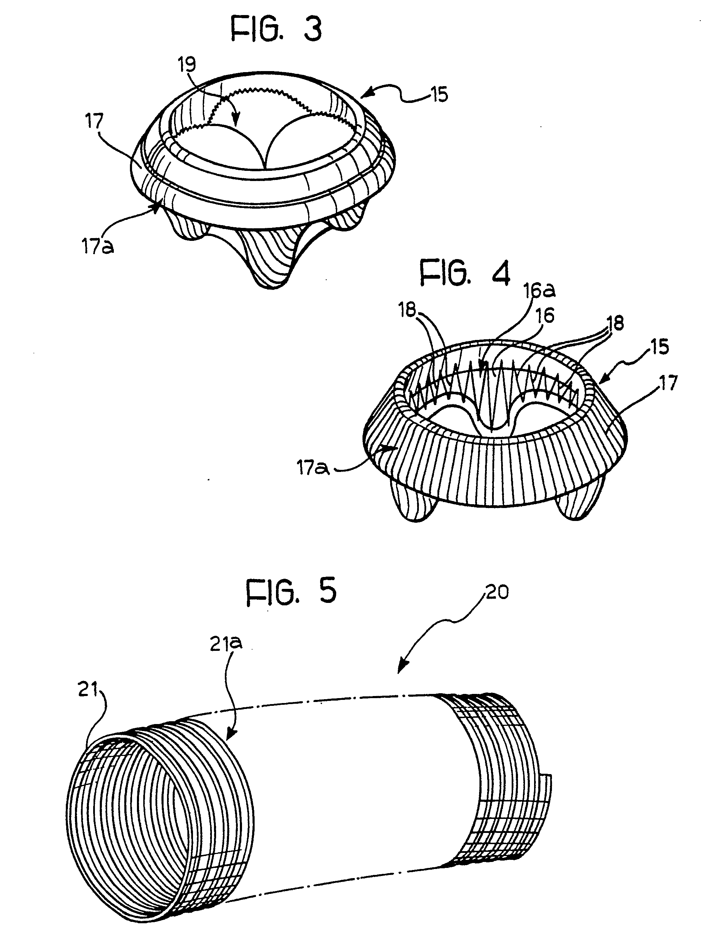

In Figure 3 a cardiac valve prosthesis is generally indicated 15 and comprises an armature 16 (Figure 4) of polymeric material such as polytetrafluoroethylene ("Teflon") of a polyacetal resin ("Delrin").

The armature 16 is covered with a synthetic yarn textile 17 to enable the prosthesis 15 to be fixed in the implant position.

The cover 17 is fixed to the armature 16 by suturing 18 with polymeric thread, for example surgical thread.

Within the armature 16 is a valve sleeve generally indicated 19 comprising membranous layers of biological material such as bovine or swine pericardium tissue.

The membranous layers of the valve sleeve 19 are connected together so as to form valve membranes adapted to carry out a function similar to the function of the valve membranes of the natural valve replaced by the prosthesis 15.

The sleeve 19 may be fixed to the armature 16 by locking rings or further suturing with polymeric thread.

The surfaces of the armature 16 and the cover 17 are coated with layers of biocompatible carbonaceous material indicated 16a and 17a respectively.

A similar coating layer may be applied to the suture threads 18 which connect the cover to the armature.

As indicated above, the coating layers 16a and 17a substantially improve the biocompatibility and resistance to the formation of thrombi of the prosthesis 15.

The coating of carbonaceous material is applied by the armature 16, possibly with the cover 17 already connected thereto by means of the suture threads 18, being placed within a cathode sputtering device of the type illustrated in Figure 1 or in Figure 2.

In this device, the covered armature 16 is coated with a protective layer of biocompatible carbonaceous material without this resulting in overheating of the armature or of the cover which are formed of materials having rather low melting points.

Alternatively it is possible to coat the armature 16, the cover 17 and the thread 18 separately, before assembly of the prosthesis 15.

With reference to the device of Figure 1, it should also be noted that the temperature of the prosthetic devices may be controlled by means of the cooling circuit provided in the support 5.

In the valvular prosthesis illustrated in Figures 3 and 4, both the armature 16 itself and its cover 17 are formed of polymeric material. Clearly, however, the invention may also be applied to cardiac valve prosthesis in which the armature 16 is made of metal and only the sutured cover 17 is formed of polymeric material.

Finally it should be noted that, by using cathode sputtering, it is possible to apply the coating of biocompatible carbonaceous material only to those parts of the prosthesis 15 which will come into direct contact with a blood flow.

As a further example of application of the invention, Figure 5 shows schematically a vascular prosthesis 20 which can be substituted for a section of natural tubing.

The prosthesis 20 comprises a tubular body 21 constituted by a textile woven from polymeric fibres such as polytetrafluoroethylene ("Teflon"), the material known commercially as "Dacron", or the like.

The wall of the tubular body 21 normally has circumferential corrugations intended to improve the longitudinal flexibility of the prosthesis 20 itself.

The tubular body of woven polymer may be located within a cathode sputtering device, such as the device illustrated in Figure 1 or Figure 2, and a layer of biocompatible carbonaceous material 21a may be deposited thereon so as to protect the inner face of the tubular body 21 which is intended to come into direct contact with the blood. Alternatively the tubular body 21 may be completely coated with the biocompatible carbonaceous material.

**Claims for the Contracting States: AT BE CH FR GB IT LI LU NL SE**

1. Method of manufacturing a prosthetic device including an element having a coating of biocompatible carbonaceous material applied by cathode sputtering, characterised in that:
— said element is chosen of a flexible polymeric material, and
— cathode sputtering of said coating is effected using a target material (2) selected from the group consisting of graphite, glassy carbon and carbon with a turbostratic structure and by means of ionization of an inert gas.

2. Method according to Claim 1, characterised in that the gas which is ionized is constituted essentially of argon.

**Claims for the Contracting State: DE**

1. Method of manufacturing a prosthetic device including an element having a coating of biocompatible carbonaceous material applied by cathode sputtering, wherein said element is chosen of a flexible polymeric material, and cathode sputtering of said coating is effected by means of ionization of an inert gas, characterised in that a target material (2) is used selected from the group consisting of graphite, glassy carbon and carbon with a turbostratic structure.

2. Method according to Claim 1, characterised in that the gas which is ionized is constituted essentially of argon.

**Patentansprüche für die Vertragsstaaten: AT BE CH FR GB IT LI LU NL SE**

1. Verfahren zur Herstellung einer Prothese mit einem Element, welches einen · durch Kathodenzerstäubung aufgetragenen Mantel aus biokompatiblem kohlenstoffhaltigem Material aufweist, dadurch gekennzeichnet, daß
— das Element aus flexiblem polymerem Material gewählt wird, und daß
— die Kathodenzerstäubung des Mantels unter Verwendung eines Auffangmaterials (2), welches aus der Gruppe von Graphit, glasartigem Kohlenstoff und Kohlenstoff mit turbostratischer Struktur ausgewählt ist, und durch Ionisierung eines inerten Gases durchgeführt wird.

2. Verfahren nach Anspruch 1, dadurch gekennzeichnet, daß das ionisierte Gas im wesentlichen aus Argon besteht.

**Patentansprüche für den Vertragsstaat: DE**

1. Verfahren zur Herstellung einer Prothese mit einem Element, welches einen durch Kathodenzerstäubung aufgetragenen Mantel aus biokompatiblem kohlenstoffhaltigem Material aufweist, wobei das Element aus flexiblem polymerem Material gewählt wird und die Kathodenwnzerstäubung des Mantels durch Ionisierung eines inerten Gases bewirkt wird, dadurch gekennzeichnet, daß ein Auffangmaterial (2) verwendet wird, welches aus der Gruppe von Graphit, glasartigem Kohlenstoff und Kohlenstoff mit turbostratischer Struktur ausgewählt ist.

2. Verfahren nach Anspruch 1, dadurch gekennzeichnet, daß das inonisierte Gas im wesentlichen aus Argon besteht.

**Revendications pour les Etats contractants: AT BE CH FR GB IT LI LU NL SE**

1. Procédé de fabrication d'un dispositif de prothèse comportant un élément qui porte un revêtement de matériau carboné biocompatible appliqué par pulvérisation cathodique, caractérisé en ce que:
— ledit élément est choisi en matériau polymère souple, et
— la pulvérisation cathodique dudit revêtement est effectuée en utilisant un matériau cible (2) choisi dans le groupe comprenant le graphite, le carbone vitreux et le carbone ayant une structure turbostratique, et par ionisation d'un gaz inerte.

2. Procédé selon la revendication 1, caractérisé en ce que le gaz qui est ionisé est constitué essentiellement d'argon.

**Revendications pour l'Etat contractant: DE**

1. Procédé de fabrication d'un dispositif de prothèse comprenant un élément qui porte un revêtement de matériau carboné biocompatible appliqué par pulvérisation cathodique, dans lequel ledit élément est choisi en matériau polymère souple, et la pulvérisation cathodique dudit revêtement est effectuée par ionisation d'un gaz inerte, caractérisé en ce qu'on utilise un matériau cible (2) choisi dans le groupe comprenant le graphite, le carbone vitreux et le carbone ayant une structure turbostratique.

2. Procédé selon la revendication 1, caractérisé en ce que le gaz qui est ionisé est constitué essentiellement d'argon.

0 102 328

FIG. 1

FIG. 2

1

## FIG. 3

19

17

17a

15

## FIG. 4

18  16a  16  18

15

17

17a

## FIG. 5

20

21a

21